# EUROPÄISCHE PATENTANMELDUNG

(11) **EP 0 562 373 A2**
(43) Veröffentlichungstag der Anmeldung: **29.09.1993**
(21) Anmeldenummer: 93103888.9
(22) Anmeldetag: 10.03.1993
(51) Int. Cl.: C12N 11/08

(54) **Immobilisierung biochemischer Substanzen**

(30) Priorität: 23.03.1992 DE 4209369
(71) Anmelder: SIEMENS AKTIENGESELLSCHAFT, D-80333 München (DE)
(72) Erfinder: von Gentzkow, Wolfgang, Dr., W-8524 Kleinsendelbach (DE); Feucht, Hans-Dieter, Dr., W-7253 Renningen (DE)

(57) **Zusammenfassung**

Ein Verfahren zur Immobilisierung von biochemischen Substanzen, insbesondere Enzyme, ist durch folgende Schritte gekennzeichnet:
- Ein olefinisch ungesättigter, epoxyfunktioneller Polyether wird in Form einer Schicht auf ein Trägermaterial aufgebracht,
- der Polyether wird durch energiereiche Strahlung oder peroxidisch zu einer weitmaschigen epoxyfunktionellen Polymermatrix vernetzt,
- die Schicht wird mit einer wäßrigen Lösung der biochemischen Substanz behandelt, wobei die biochemische Substanz durch Reaktion mit Epoxidgruppen in der Polymermatrix immobilisiert wird,
- die Schicht wird durch Reaktion von nicht umgesetzten Epoxidgruppen mit einer amino- und/oder carboxylgruppenhaltigen Verbindung stabilisiert.

## Beschreibung

Die Erfindung betrifft ein Verfahren zur Immobilisierung von biochemischen Substanzen, insbesondere Enzyme.

Enzyme werden - wegen ihrer Selektivität und hohen katalytischen Aktivität - in zunehmendem Maße in vielen Bereichen der Lebensmittel-, Pharma- und chemischen Industrie für die Herstellung und Analyse von Produkten sowie in der Medizin zur Diagnostik und Therapie eingesetzt. Obwohl die Enzyme bei den durch sie katalysierten Umsetzungen nicht verbraucht werden, sind sie wegen ihrer Substratlöslichkeit nicht wiederverwendbar. Dies bringt eine Reihe von Nachteilen mit sich, die man seit einiger Zeit durch den Einsatz immobilisierter Enzyme zu überwinden versucht.

Unter Immobilisierung wird die Überführung der wasserlöslichen Enzyme - unter Erhaltung der katalytischen Wirksamkeit - in eine wasserunlösliche Form verstanden. Dies ist durch chemische und/oder physikalische Bindung der wasserlöslichen Enzyme an einen wasserunlöslichen Träger sowie durch Einschluß in wasserunlösliche Gelmatrizes oder Mikrokapseln möglich. Der Einsatz immobilisierter Enzyme ist grundsätzlich auf Prozesse mit wäßrigen oder wasserhaltigen flüssigen Substraten beschränkt. Die wesentlichen Vorteile immobilisierter Enzyme bestehen in der leichten Abtrennbarkeit von der Reaktionslösung und in der Wiederverwendbarkeit. Diese Vorteile bedeuten vor allem bei aufwendig und in geringer Ausbeute zugänglichen Enzymen eine erhebliche Kostenersparnis. Da die Endprodukte frei von Enzymen bleiben, entfällt auch die sonst erforderliche Hitzebehandlung zur Inaktivierung gelöster Enzyme, was sich im Falle hitzeempfindlicher Produkte als besonders vorteilhaft erweist. Hinzu kommt noch, daß bei der Verwendung immobilisierter Enzyme eine kontinuierliche Prozeßführung mit präziser Prozeßkontrolle möglich ist.

Jedes Verfahren mit immobilisierten Enzymen steht in Konkurrenz mit dem gleichen Verfahren mit gelösten Enzymen. Immobilisierte Enzyme sind dabei nur dann konkurrenzfähig, wenn durch ihren Einsatz klare wirtschaftliche Vorteile erreicht werden, beispielsweise indem verbesserte und reinere Produkte erhalten werden, die einfacher, schneller und kostengünstiger aufgearbeitet werden können.

Zur Immobilisierung von Enzymen sind bislang folgende Methoden beschrieben worden:
- Adsorption
- ionische Bindung
- Absorption
- kovalente Bindung an eine Trägeroberfläche
- Einschluß in eine Matrix oder in Mikrokapseln
- Einschluß durch Umhüllung mit einer Membran (Makroverkapselung)
- Quervernetzung bzw. Copolymerisation mit di- oder polyfunktionellen Monomeren.
Alle diese Methoden sind aber nicht universell anwendbar. Nur wenn die Anwendung eines Enzyms genau definiert ist, können ein passender Träger, die Immobilisierungsmethode und die Reaktorform ausgewählt und aufeinander abgestimmt werden (siehe dazu beispielsweise: W. Hartmeier, "Immobilisierte Biokatalysatoren", Springer-Verlag Berlin, Heidelberg 1986, Seiten 23 bis 51, und J. Woodward, "Immobilised cells and enzymes", IRL Press, Oxford, Washington DC, 1985, Seiten 3 bis 54, sowie W. Crueger und A. Crueger, "Biotechnologie - Lehrbuch der angewandten Mikrobiologie", R. Oldenbourg Verlag München, Wien 1989, Seiten 201 bis 203).

Die physikalische Adsorption eines Enzyms an einen wasserunlöslichen Träger ist die einfachste und älteste Methode zur Immobilisierung von Enzymen. Sie beruht auf nicht-spezifischen physikalischen Wechselwirkungen zwischen dem Enzymprotein und der Oberfläche des Trägermaterials. Die Bindungskräfte sind dabei hauptsächlich Wasserstoffbrücken und van der Waalssche Kräfte (siehe dazu: S.A. Barker and I. Kay in "Handbook of Enzyme Biotechnology" (Editor: A. Wiseman), Ellis Horwood, Chichester 1975, Kap. 5, Seite 89). Zur Immobilisierung wird eine konzentrierte Enzymlösung mit dem Trägermaterial gemischt. Häufig verwendete Trägermaterialien sind Aktivkohle, Aluminiumoxid, Siliciumdioxid, poröses Glas, Cellulose und phenolische Kunstharze.

Die Adsorption hat den Nachteil, daß es - aufgrund der schwachen Bindungskräfte - durch Änderung der Temperatur, des pH-Wertes oder der Ionenstärke sowie durch die Anwesenheit anderer Stoffe in der Reaktionslösung im Verlauf der Anwendung zur Desorption des Enzyms kommt. Ein weiterer Nachteil ist, daß die Adsorption nicht spezifisch ist und somit weitere Proteine oder andere Substanzen aus der Reaktionslösung adsorbiert werden können. Dadurch können die Eigenschaften des immobilisierten Enzyms verändert werden und Aktivitätsverluste auftreten.

Bei der ionischen Bindung wird das elektrostatisch geladene Enzymmolekül von einem entgegengesetzt geladenen polyanionischen oder polykationischen Träger angezogen und fixiert.

Wie bei der Adsorption kommt es auch hierbei nur zu einer relativ schwachen Bindung, da die Ladung des Enzymproteins, bezogen auf dessen Masse, sehr klein ist. Auch ist die Anwendung dieser Methode nur bei geringen Salzgehalten der Substratlösung möglich, da andere stärkere Ionen bei Anwesenheit im Substrat die Enzymmoleküle leicht verdrängen können. Die am meisten verwendeten Ionenaustauscherharze sind DEAE-Cellulose (DEAE = Diethylaminoethyl), DEAE-Sephadex (ein Agarose-Präparat) und CM-Cellulose (CM = Carboxymethyl). Auch bei der Absorption in Polymerschichten werden relativ instabile Systeme erhalten. Migration und Extraktion der Enzyme führen dabei zu einer ständigen Aktivitätsabnahme und begrenzen die Lebensdauer der Enzymschicht.

Wesentlich stabilere Systeme werden erhalten, wenn die Enzyme kovalent an eine Trägeroberfläche gebunden, über Quervernetzung bzw. Copolymerisation unlöslich gemacht oder durch Mikro- bzw. Makroverkapselung immobilisiert werden. Für die Bildung kovalenter Bindungen und die Quervernetzung stehen seitens der Enzyme freie Amino-, Carboxyl-, Hydroxyl- und Mercaptogruppen zur Verfügung. Als Trägermaterial können sowohl anorganische Materialien, wie Glas, als auch natürliche und synthetische organische Polymere verwendet werden. Voraussetzung ist dabei, daß die Trägermaterialien reaktive Gruppen, wie Isocyanat-, Isothiocyanat-, Säurechlorid- und Epoxidgruppen, enthalten. Weniger reaktive Gruppen lassen sich aktivieren, beispielsweise Carboxylgruppen durch das Carbodiimid- oder Azidverfahren, Hydroxylgruppen durch die Bromcyanmethode und Aminogruppen durch das Isothiocyanat- oder Azoverfahren. Vor allem auf der Basis von Acryl- und Methacrylsäurederivaten gelang es, zahlreiche reaktive Copolymerisate mit Dinitrofluorphenyl-, Isothiocyanat-, Oxiran- oder Säureanhydridgruppen herzustellen. Kommerziell erhältlich sind beispielsweise Polyacrylamide mit Oxirangruppen sowie modifizierte Copolymerisate auf der Basis von Vinylacetat und Divinylethylenharnstoff mit Oxirangruppen.

Die Immobilisierung durch Quervernetzung bzw. durch Copolymerisation stellen Sonderformen der kovalenten Bindung dar. Bei diesen Methoden erfolgt die Ausbildung kovalenter Bindungen zwischen den Enzymmolekülen und di- oder polyfunktionellen Monomeren, wie Glutardialdehyd, bei der Copolymerisation zusätzlich zwischen den Enzymmolekülen und einer polymerisierenden Substanz. Auf diese Weise entstehen hochmolekulare unlösliche Aggregate. Die Quervernetzung wird als Immobilisierungsmethode im allgemeinen in Kombination mit einer der anderen Methoden angewendet, beispielsweise in Kombination mit der Adsorption oder der Absorption. Hierbei werden die Enzymmoleküle zunächst an der Oberfläche des Trägers adsorbiert oder in einer darauf befindlichen Schicht absorbiert und anschließend miteinander vernetzt.

Ein wesentlicher Nachteil der Immobilisierung durch kovalente Bindung ist die damit verbundene starke Belastung der Biokatalysatoren. Die erforderlichen, zum Teil rauhen Immobilisierungsprozeduren, bei denen ein starker Wechsel des pH-Wertes auftritt, organische Lösungsmittel verwendet werden müssen oder Umsetzungen mit niedermolekularen reaktiven Substanzen erfolgen, führen nämlich fast immer zu starken Konformationsänderungen und damit zu Aktivitätsverlusten derart gebundener Enzyme.

Bei der Immobilisierung durch Einschluß, d.h. Mikro- oder Makroverkapselung, werden nicht die Enzyme selbst unlöslich gemacht, sondern ihr Reaktionsbereich wird durch semipermeable Polymere bzw. Polymerschichten begrenzt. Voraussetzung für die Funktionstüchtigkeit der in dieser Weise eingehüllten Enzyme ist, daß Substrate und Produkte die Hüllsubstanz passieren können, während die Enzyme selbst zurückgehalten werden müssen. Für die Matrixeinhüllung werden neben natürlichen Polymeren, wie Alginat, Carrageenan, Pektin, Agar und Gelatine, die allerdings für eine dauerhafte Immobilisierung von Enzymen zu grobmaschig sind, insbesondere synthetische Polymere, wie Polyacrylamid, verwendet. Zur Verkapselung dienen beispielsweise Polyamide, Polyurethane, Polyester und Polyharnstoffe. Die Einschlußmethode hat den Nachteil, daß relativ dicke Schichten mit langen Diffusionswegen entstehen.

Aufgabe der Erfindung ist es, ein Verfahren zur Immobilisierung von biochemischen Substanzen anzugeben, das technisch einfach, verlustarm, rationell und kostengünstig durchführbar ist und insbesondere reproduzierbar funktionsstabile und katalytisch ausreichend aktive immobilisierte Enzyme liefert.

Dies wird erfindungsgemäß dadurch erreicht, daß ein olefinisch ungesättigter, epoxyfunktioneller Polyether in Form einer Schicht auf ein Trägermaterial aufgebracht wird, daß der Polyether durch energiereiche Strahlung oder peroxidisch zu einer weitmaschigen epoxyfunktionellen Polymermatrix vernetzt wird, daß die Schicht mit einer wäßrigen Lösung der biochemischen Substanz behandelt wird, wobei die biochemische Substanz durch Reaktion mit Epoxidgruppen in der Polymermatrix immobilisiert wird, und daß die Schicht durch Reaktion von nicht umgesetzten Epoxidgruppen mit einer amino- und/oder carboxylgruppenhaltigen Verbindung stabilisiert wird.

Die Problemlösung besteht bei der Erfindung somit in einem neuen Verfahren zur Immobilisierung von Enzymen und anderen biochemischen Substanzen, und zwar in vernetzten epoxyfunktionellen Polyethern. Es wurde nämlich überraschenderweise gefunden, daß diese Substanzen - aus wäßriger Lösung - in weitmaschig vernetzte epoxyfunktionelle Polyether einzudringen vermögen und durch Reaktion mit kettenständigen Epoxidgruppen unter sehr milden Bedingungen in der Polymermatrix, d.h. im Polymernetzwerk, verankert werden können. Diese Tatsache ist völlig neu, und sie eröffnet die Möglichkeit einer sehr schonenden Immobilisierung. Das erfindungsgemäße Verfahren ist nicht auf die Immobilisierung von Enzymen beschränkt, sondern es ermöglicht auch die Immobilisierung von anderen biochemischen Substanzen, wie Coenzyme, Enzyminhibitoren, Effektoren und Antikörper.

Bevorzugte Ausführungsformen des Verfahrens nach der Erfindung sind Gegenstand von Unteransprüchen.

Das erfindungsgemäße Verfahren beinhaltet im allgemeinen folgende Schritte:
1. Beschichtung von Trägermaterialien
   Ein radikalisch vernetzbarer, epoxyfunktioneller Polyether oder eine Mischung derartiger Polyether wird, gegebenenfalls in Kombination mit einem Vernetzungsinitiator, einem Vernetzungsverstärker und/oder weiteren Zusatzstoffen, in der gewünschten Schichtdicke auf ein Trägermaterial aufgebracht. Der Träger kann aus anorganischem oder organischem Material bestehen und in Form von Fasern, Vliesen, Papier und Geweben oder in Form von Flächenstoffen vorliegen. Als besonders günstig kann sich die Verwendung von porösen oder porenfreien Membranmaterialien erweisen. Je nach Anwendungsfall und Fließverhalten des verwendeten Polyethers kann die Beschichtung aus einer Lösung oder lösungsmittelfrei durch Tauchen, Spin-Coating, Roller-Coating, Curtain-Coating oder einen anderen in der Technik üblichen Prozeß erfolgen, wobei die Vorbehandlung der Trägeroberfläche mit einem Haftvermittler erforderlich sein kann. Bei langgestrecktem Gut kann kontinuierlich beschichtet werden. Die Schichtdicke kann über die Einstellung der Viskosität und durch Zusatz eines Lösemittels oder eines Reaktivverdünners gesteuert werden. Die auf diese Weise hergestellte Schicht muß in jedem Fall von flüchtigen Bestandteilen befreit werden, was beispielsweise durch Trocknen oder Entgasen erfolgen kann. Zur verbesserten Haftung der Beschichtung auf der gegebenenfalls mit einem Haftvermittler vorbehandelten Trägeroberfläche kann sich ein Temperschritt als vorteilhaft erweisen.
2. Vernetzen der Beschichtung
   Die Vernetzung der Beschichtung, d.h. des Polyethers, erfolgt durch energiereiche Strahlung, insbesondere durch UV-, Elektronen- und γ-Strahlen, oder peroxidisch. Zur peroxidischen Vernetzung werden in den Polyether bzw. in die Polyethermischung organische Peroxide eingebracht. Die Vernetzung wird durch thermischen Zerfall der Peroxide in Radikale initiiert. Die Wärmeübertragung auf die Beschichtung kann durch IR-Strahlung, Mikrowellen, geheizte Walz- und Preßwerkzeuge oder durch heiße Gase erfolgen. Durch die Vernetzung, bei der ausschließlich die olefinisch ungesättigten, radikalisch polymerisierbaren Gruppen umgesetzt werden, während die Epoxidgruppen quantitativ erhalten bleiben, entsteht ein weitmaschiges Polymernetzwerk.
3. Immobilisierung der biochemischen Substanz
   Beim Kontaktieren der vernetzten Schicht mit einer wäßrigen Lösung der biochemischen Substanz wandert diese Substanz in die Polymermatrix hinein und wird dort durch Reaktion mit den Epoxidgruppen kovalent gebunden. Voraussetzung für diesen Prozeß ist - neben der erforderlichen Maschenweite - eine ausreichende Hydrophilie des bei der Vernetzung gebildeten Polymernetzwerkes. Die Immobilisierung kann deshalb durch eine vorangehende Hydrophilierung des Polyethers beschleunigt werden. Dies erfolgt durch Umsetzung eines Teils der Epoxidgruppen mit hydrophilen Verbindungen, die reaktive Gruppen, wie NH-, OH-, SH- oder COOH-Gruppen, enthalten, womit der hydrophile Charakter der Polymerschicht erhöht wird. Der Immobilisierungsprozeß kann auch durch Zusatzstoffe, wie Polyvinylpyrrolidon, die eine erhöhte Wasseraufnahme der Polyether bewirken, sowie durch mit Wasser mischbare Lösemittel, wie Dioxan, Tetrahydrofuran, Alkohole oder Polyether, erheblich beschleunigt werden. Im übrigen können in einer Schicht auch mehrere unterschiedliche biochemische Substanzen immobilisiert werden, was gleichzeitig oder nacheinander erfolgen kann.
4. Stabilisierung der Beschichtung
   Dieser Schritt beinhaltet die Reaktion von nach der Immobilisierung verbliebenen Epoxidgruppen mit einer amino- und/oder carboxylgruppenhaltigen Verbindung, insbesondere einer Aminosäure. Die Stabilisierung kann - in Abhängigkeit von der verwendeten Verbindung - zu einer engeren Vernetzung der Schicht und damit zu einer verbesserten mechanischen Festigkeit oder zur Anpassung der Materialeigenschaften und des Stofftransports genutzt werden. Im übrigen ist eine oberflächliche Abdeckung dieser Schicht durch eine oder mehrere zusätzliche Schichten möglich, was auch zur Einstellung definierter Diffusionsbedingungen der umzusetzenden Reaktionspartner und -produkte sinnvoll sein kann.

Für das erfindungsgemäße Verfahren eignen sich insbesondere epoxyfunktionelle Polyether folgender Struktur, die Gegenstand der gleichzeitig eingereichten europäischen Patentanmeldung Nr. - "Polyether" (GR 92 P 3135 E) sind:

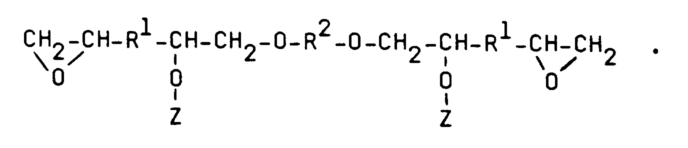

Dabei gilt folgendes:
Z = 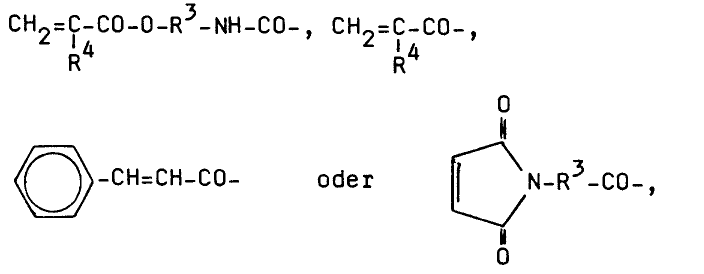 wobei
R³ = -(CH₂)ₘ-, mit m = 1 bis 10,
R⁴ = H oder CH₃;
R¹ = -(CH₂)ₒ-, mit o = 0 bis 18,
-CH₂-O-R⁵-O-CH₂-,
wobei
R⁵ = -(CH₂)ₚ-, 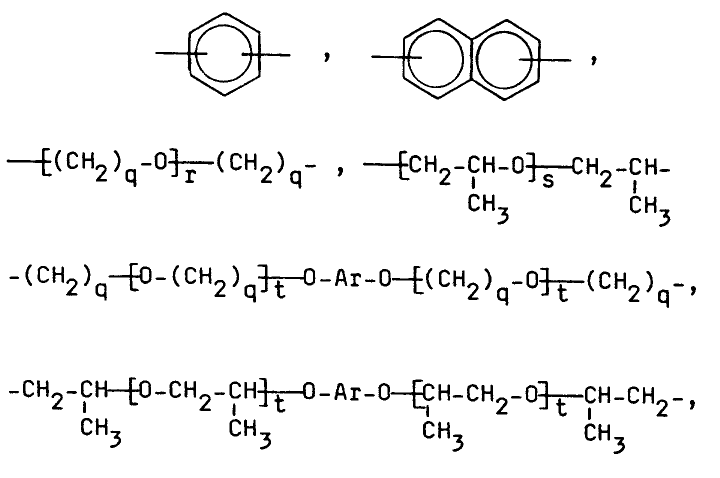 mit p = 2 bis 20, q = 2 bis 4, r = 1 bis 50,
s = 0 bis 50, t = 0 bis 25,
Ar = 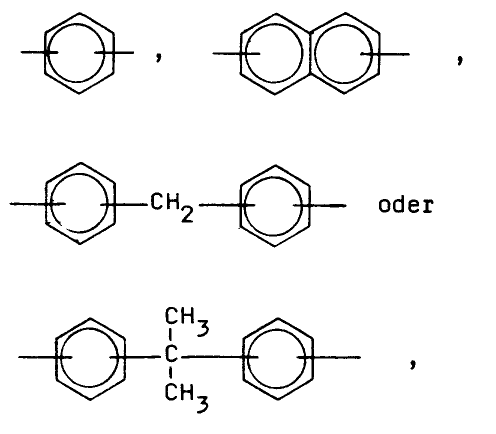 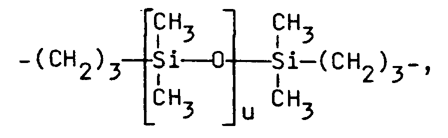 mit u = 0 bis 150,
oder die entsprechende Gruppierung aus 3,4-Epoxycyclohexylmethyl-3',4'-epoxycyclohexancarboxylat, d.h. der Verbindung: 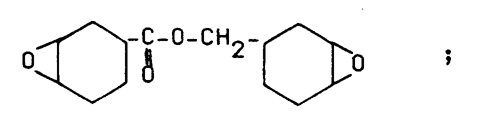
R² = -(CH₂-CH=CH-CH₂)ₙ- , -R⁶- , -R⁶-O-CO-R⁷-CO-O-R⁶- oder 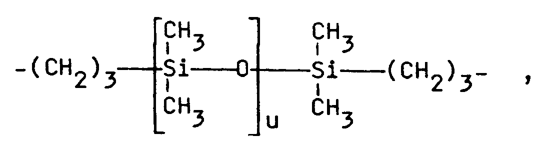 wobei n = 1 bis 50, u = 0 bis 150,
R⁶ dieselbe Bedeutung hat wie R⁵,
außer 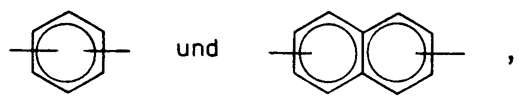 und
R⁷ folgendes bedeutet:
-(CH₂)ᵥ-, 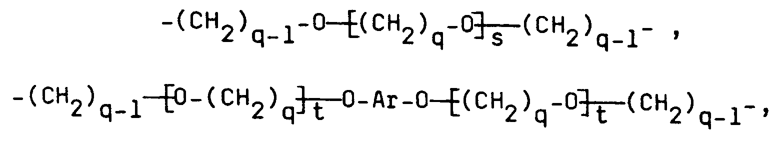 mit q = 2 bis 4, s = 0 bis 50, t = 0 bis 25,
v = 0 bis 20, und
Ar die vorstehend angegebene Bedeutung hat.

Das erfindungsgemäße Verfahren bietet folgende Vorteile:
- Es erlaubt die Immobilisierung aller biochemischen Substanzen, die an der Peripherie über reaktive NH-, OH-, SH- oder COOH-Gruppen verfügen.
- Nach diesem Verfahren hergestellte Schichten mit immobilisierten biochemischen Substanzen können auch trocken und unter nicht-sterilen Bedingungen ohne Schädigung dieser Substanzen aufbewahrt werden.
- Die Immobilisierung der biochemischen Substanzen erfolgt unter sehr milden Bedingungen in wäßriger Lösung und in Abwesenheit niedermolekularer reaktiver Verbindungen; dadurch werden Verluste, beispielsweise durch Enzymdenaturierung, vermieden.
- Für die Immobilisierung einer großen Zahl verschiedenartiger biochemischer Substanzen dient eine relativ kleine Zahl großtechnisch herstellbarer und damit kostengünstig zugänglicher Polymermaterialien hoher chemischer und thermischer Stabilität.
- Die Herstellung und Vernetzung der Schichten kann technisch einfach, reproduzierbar und kostengünstig durchgeführt werden. Bei der Verwendung von langgestrecktem Gut als Träger, wie Folien, Gewebe, Schläuche und Bänder, können kontinuierlich arbeitende Verfahren zum Einsatz gelangen.
- Die Immobilisierung der biochemischen Substanzen kann - je nach Bedarf und Einsatzzweck - unabhängig von der Schichtherstellung erfolgen, gegebenenfalls auch erst beim Anwender, kurz vor dem Einsatz.
- Durch die chemische Verankerung der biochemischen Substanzen in der Polymermatrix werden Desorptions-, Migrations- und Extraktionsverluste vermieden.
- Durch die Ausbildung kovalenter Bindungen zwischen den peripheren NH-, OH-, SH- und COOH-Gruppen der biochemischen Substanzen und dem sehr weichen und flexiblen umhüllenden Polymermaterial wird den zum Teil sehr empfindlichen Substanzen, beispielsweise Enzyme, eine hohe Funktions- und Langzeitstabilität verliehen.
- Die Herstellung sehr dünner Schichten (« 1 µm) ermöglicht sehr kurze Diffusionswege für Reaktionspartner und -produkte.
- Da beim erfindungsgemäßen Verfahren Größe, Form, Hydrophilie und Reaktivität der zu immobilisierenden biochemischen Substanzen eine große Rolle spielen, ist die Immobilisierung mit einer gewissen Selektion und Reinigung verbunden; dies ermöglicht in vielen Fällen den Einsatz kostengünstiger Produkte mit niedriger Aktivität.

Das erfindungsgemäße Verfahren kann überall dort technisch genutzt werden, wo heutzutage bereits immobilisierte biochemische Substanzen verwendet werden bzw. wo diese vorteilhaft eingesetzt werden können. Besondere Vorteile bietet dieses Verfahren beim Einsatz in Enzymreaktoren, wie sie beispielsweise zur Herstellung von L-Aminosäuren aus Acetyl-DL-aminosäuren, α-Ketocarbonsäuren, α-Hydroxycarbonsäuren oder α,β-ungesättigten Carbonsäuren, zur Herstellung von L-Äpfelsäure aus Fumarsäure, zur Isomerisierung von Glucose sowie zur Penicillin-Derivatisierung in technischem Maßstab genutzt werden. Dabei kann die Immobilisierung der benötigten Enzyme in dünnen Schichten auf sehr unterschiedlichen Materialien erfolgen. Neben verschiedenen Metallen und Metalloxiden kommt dafür insbesondere eine Vielzahl verschiedener Kunststoffe in Frage. Besondere Vorteile bietet die Immobilisierung in Schichten auf porösen Membranen für den Einsatz in Membranreaktoren.

Vorteile bringt die Anwendung des erfindungsgemäßen Verfahrens auch bei der Identifizierung, Trennung und Reinigung biochemischer Stoffe. In der Analytik bietet insbesondere der Einsatz im Rahmen der Affinitätschromatographie interessante Anwendungsmöglichkeiten. In der Medizin ist das erfindungsgemäße Verfahren für die intra- und extrakorporale Enzymtherapie und zur Herstellung künstlicher Organe, beispielsweise von künstlichen Nieren, einsetzbar. Durch Immobilisierung bestimmter biochemischer Substanzen, wie Heparin, kann die Bioverträglichkeit der Polyetherschichten erhöht werden, so daß diese beispielsweise als Beschichtung für Implantate dienen können.

Anhand von Ausführungsbeispielen soll die Erfindung noch näher erläutert werden.

### Beispiel 1

### Herstellung von Polyether/Enzym-Schichten

100 Masseteile eines epoxyfunktionellen Polyethers der Struktur 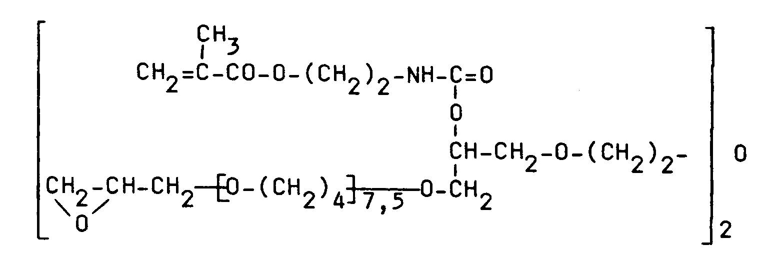
werden mit 7 Masseteilen propoxyliertem Glycerintriacrylat als Reaktivverdünner und 2 Masseteilen 2-Hydroxy-2-methyl-1-phenylpropanl-1-on als Photoinitiator gemischt und zur Einstellung der benötigten Verarbeitungseigenschaften mit der entsprechenden Menge Toluol versetzt. Diese Lösung wird dann durch Tauchen, Tropfen oder Rakeln auf die gegebenenfalls mit einem Haftvermittler vorbehandelte Oberfläche des Trägermaterials aufgebracht. Parallel dazu werden mit der gleichen Lösung Siliciumwafer mit Hilfe einer Lackschleuder beschichtet; die Schleuderzeit beträgt ca. 10 s.

Die Schichten werden in einer Laminarbox getrocknet und danach unter Stickstoff durch UV-Bestrahlung (Anlage F 450 der Fa. Fusion UV-Curing Systems) im Wellenlängenbereich 200 bis 450 nm vernetzt; Bestrahlungsdauer: 4,6 s. Zur Entfernung löslicher Anteile werden die vernetzten Schichten bei Raumtemperatur 24 h mit Dioxan extrahiert. Zur Erhöhung der Hydrophilie der Schichten wird ein Teil der Epoxidgruppen mit NH-Gruppen-haltigen Verbindungen in Form von Aminosäuren umgesetzt. Dabei hat sich insbesondere eine Lagerung der Schichten in einer 2 %igen Lösung von Prolin oder Glutaminsäure in einer 2:1-Mischung aus Dioxan und Wasser bei 40 bis 60°C bewährt. Anhand parallel behandelter Siliciumwafer kann der Umsatz IR-spektroskopisch verfolgt werden. Ein Umsatz von 50 % ist in den meisten Fällen ausreichend; bei Bedarf können aber auch höhere Werte eingestellt werden.

Die Immobilisierung der Enzyme erfolgt durch Inkubation der Schichten in einer ca. 1 bis 2 %igen Lösung des Enzyms in Wasser bei 20 bis 30°C. Zur Beschleunigung dieses Vorgangs kann die Lösung, je nach Empfindlichkeit des Enzyms, mit 10 bis 50 % Dioxan versetzt werden. Die Immobilisierung ist nach 1 bis 8 h beendet. Verbliebene Epoxidgruppen können durch schonende Umsetzung mit Aminosäuren beseitigt werden. Zum Abschluß werden die Schichten durch intensives Waschen mit Wasser von extrahierbaren Anteilen befreit.

Tabelle 1 enthält eine Zusammenstellung der erfindungsgemäß in 10 µm dicken, identisch vorbehandelten Schichten auf Siliciumwafern bei 30°C innerhalb von 8 h immobilisierten Enzyme sowie die Enzymaktivität bei 25°C.

### Beispiel 2

### Einfluß der Enzymaktivität auf die Aktivität von Polyether/Enzym-Schichten

Eine gemäß Beispiel 1 auf einem Filterpapier erzeugte ca. 20 µm dicke Polyetherschicht wird nach der UV-Vernetzung und der Extraktion mit Dioxan in einer 2 %igen Lösung von Prolin in einer 2:1-Mischung aus Dioxan und Wasser 6 h bei 60°C inkubiert. Das Filterpapier wird geteilt, und die eine Hälfte wird mit einer 2 %igen wäßrigen Lösung von Glucoseoxidase einer Aktivität von 22 U/mg und die andere Hälfte mit einer 2 %igen wäßrigen Lösung von Glucoseoxidase einer Aktivität von 276 U/mg jeweils 8 h bei 30°C behandelt. Beide Hälften werden dann getrennt 24 h gewässert. Danach werden jeweils

**Tabelle 1**

| Enzym | Aktivität | Bestimmungsmethode |
|---|---|---|
| Glucoseoxidase aus Aspergillus niger, lyophil. 240 U/mg | 0,8 U/cm² | Gluc-DH-Methode der Fa. Merck |
| Katalase aus Rinder-leber, Suspension 65 000 U/mg | 350 U/cm² | Siehe: B.Stellmach, "Bestimmungsmethoden Enzyme", Steinkopff-Verlag, Darmstadt 1988, Seiten 152 bis 155 |
| Urease aus Schwert-bohnen, lyophil. 100 U/mg | 0,7 U/cm² | Siehe: B. Stellmach, "Bestimmungsmethoden Enzyme" Steinkopff-Verlag, Darmstadt 1988, Seiten 269 bis 271 |
| Alkoholdehydrogenase aus Hefe, lyophil. 400 U/mg | 2,0 U/cm² | Siehe: B. Stellmach, "Bestimmungsmethoden Enzyme", Steinkopff-Verlag, Darmstadt 1988, Seiten 11 und 12 |
| L-Asparaginase, 50 %ige Lösung in Glycerin 80 U/mg Lösung | 0,6 U/cm² | Siehe: B. Stellmach, "Bestimmungsmethoden Enzyme", Steinkopff-Verlag, Darmstadt, 1988, Seiten 63 bis 68 |

1 cm² große Proben der beiden Hälften mittels der Gluc-DH-Methode auf ihre Aktivität untersucht. Dabei wird für die erste Hälfte im Mittel eine Aktivität von 1,6 U/cm² und für die zweite Hälfte im Mittel eine Aktivität von 1,7 U/cm² ermittelt. Dieses Ergebnis zeigt, daß die Aktivität der Polyether/Enzym-Schichten nahezu unabhängig von der Aktivität des eingesetzten Enzyms ist.

### Beispiel 3

### Herstellung von Polyether-Schichten zur Immobilisierung von Enzymen auf verschiedenen Trägermaterialien

Die in Beispiel 1 beschriebene Mischung aus epoxyfunktionellem Polyether, Reaktivverdünner und Photoinitiator wird in unterschiedlichen Mengen Toluol gelöst. Die dabei erhaltenen Lösungen werden durch Tauchen (= T) oder Spin-Coating (= S) auf verschiedene, in Tabelle 2 näher beschriebene Trägermaterialien aufgebracht. Massive Materialien werden vorher oberflächlich poliert und gereinigt und gegebenenfalls nach Vorbehandlung mit einem Haftvermittler durch Spin-Coating beschichtet; die Schleuderzeit beträgt 10 s. Folien, Membranen und Vliese werden entweder auf einen massiven Träger aufgeklebt und durch Spin-Coating beschichtet oder getaucht. Gewebe werden durch Tauchen und nachfolgendes Abstreifen oder Abdrücken überschüssigen Polyethers beschichtet. Alle Schichten werden, wie in Beispiel 1 beschrieben, durch UV-Strahlung vernetzt.

Tabelle 2 enthält eine Zusammenstellung der verwendeten Materialien und Beschichtungstechniken sowie der Polyether-Schichtdicken. Die Haftung der Schichten auf dem Trägermaterial wurde durch jeweils 24stündige Lagerung und Quellung in Dioxan bestimmt. Die Schichten können, wie in Beispiel 1 beschrieben, zur Immobilisierung von Enzymen genutzt werden.

### Beispiel 4

### Beurteilung der Wirksamkeit von Polyether/Enzym-Schichten

Analog Beispiel 3 wird auf einer Polyestermembran mit einer Schichtdicke von 14 µm eine ca. 10 µm dicke Schicht des in Beispiel 1 beschriebenen Polyethers durch Spin-Coating erzeugt. Entsprechend Beispiel 1 wird dieser Verbund 6 h bei 60°C mit Prolin und danach 6 h bei 30°C mit einer wäßrigen Enzymlösung behandelt. Folgende Enzyme werden dabei immobilisiert: Fumarase, Aktivität 200 U/mg; L-Aspartase, Aktivität 5 U/mg.

Fumarase katalysiert die Umsetzung von Fumarsäure mit Wasser zu L-Äpfelsäure, L-Aspartase die Umsetzung von Fumarsäure mit Ammoniak zu L-Asparaginsäure. Da beide Enzyme auch die jeweilige Rückreaktion katalysieren, wird mit einem Überschuß der Ausgangskomponenten gearbeitet. Die Umsetzung in Gegenwart von Fumarase erfolgt bei einem pH-Wert von 7,5, die Umsetzung in Gegenwart von L-Aspartase bei einem pH-Wert von 8,5. Die Abnahme der Fumarsäurekonzentration wird jodometrisch bestimmt (siehe dazu: "Chem. Ber.", Bd. 70 (1937), Seiten 903 bis 907).

Die beiden Umsetzungen werden in der Weise durchgeführt, daß das eine Mal ca. 10 cm² der Aspartase-haltigen Membran in 200 ml einer Lösung von 2 % Fumarsäure und 2 % Ammoniak (NH₄OH) und das andere Mal ca. 1 cm² der Fumarase-haltigen Membran in 400 ml einer 2 %igen Fumarsäurelösung eingebracht und die Lösungen gerührt werden. Um starke Konzentrationsänderungen

**Tabelle 2**

| Trägermaterial (* = in Scheibenform) | Haftvermittler | Beschichtungstechnik | Schichtdicke (µm) | Haftung |
|---|---|---|---|---|
| Silicium* | - | S | 0,1 - 50 | - |
| | + | S | 0,1 - 50 | + |
| Quarz* | - | S | 0,1 - 50 | - |
| | + | S | 0,1 - 50 | + |
| Epoxidharz* | - | S | 0,1 - 50 | + |
| | + | S | 0,1 - 50 | + |
| Polyetheretherketon* | - | S | 0,1 - 30 | + |
| Polysiloxanschicht auf Silicium* | - | S | 0,1 - 30 | + |
| Polyester-Membran | - | S | 0,1 - 30 | + |
| Cellulose-Membran | - | S | 0,1 - 30 | + |
| Cellulose-Filterpapier | - | T | 50 - 150 | + |
| Acetylcellulose-Membran | - | S | 0,1 - 50 | + |
| Nylon-6,6-Gewebe | - | T | 100 - 150 | + |
| Glasfaser-Gewebe | - | T | 100 - 200 | + |

zu vermeiden, wird die Reaktion jeweils nach 24 h unterbrochen, die Fumarsäurekonzentration bestimmt und die Reaktionslösung durch eine frisch zubereitete Lösung ersetzt.

Die ermittelten Umsatzkurven zeigen, daß die Aktivität der beiden Enzyme über einen Zeitraum von 5 Tagen praktisch nicht reduziert wird.

## Patentansprüche

1. Verfahren zur Immobilisierung von biochemischen Substanzen, insbesondere Enzyme, **dadurch gekennzeichnet,**
- daß ein olefinisch ungesättigter, epoxyfunktioneller Polyether in Form einer Schicht auf ein Trägermaterial aufgebracht wird,
- daß der Polyether durch energiereiche Strahlung oder peroxidisch zu einer weitmaschigen epoxyfunktionellen Polymermatrix vernetzt wird,
- daß die Schicht mit einer wäßrigen Lösung der biochemischen Substanz behandelt wird, wobei die biochemische Substanz durch Reaktion mit Epoxidgruppen in der Polymermatrix immobilisiert wird,
- und daß die Schicht durch Reaktion von nicht umgesetzten Epoxidgruppen mit einer amino- und/oder carboxylgruppenhaltigen Verbindung stabilisiert wird.

2. Verfahren nach Anspruch 1, **dadurch gekennzeichnet,** daß der Polyether nach der Vernetzung hydrophiliert wird.

3. Verfahren nach Anspruch 1 oder 2, **dadurch gekennzeichnet,** daß Polyether der Struktur 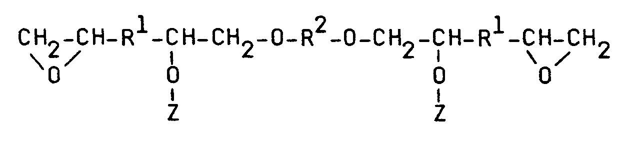 verwendet werden, wobei folgendes gilt:
Z = 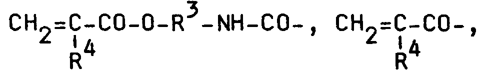 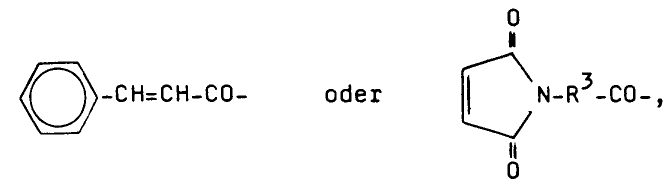 wobei
R³ = -(CH₂)ₘ-, mit m = 1 bis 10,
R⁴ = H oder CH₃;
R¹ = -(CH₂)ₒ-, mit o = 0 bis 18,
-CH₂-O-R⁵-O-CH₂-,
wobei
R⁵ = -(CH₂)ₚ-, 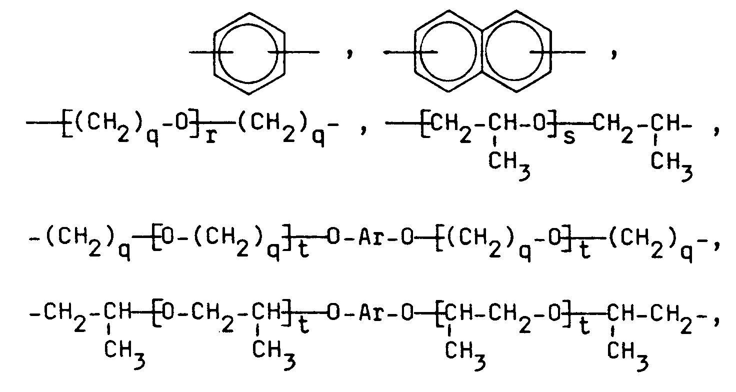 mit p = 2 bis 20, q = 2 bis 4, r = 1 bis 50,
s = 0 bis 50, t = 0 bis 25,
Ar = 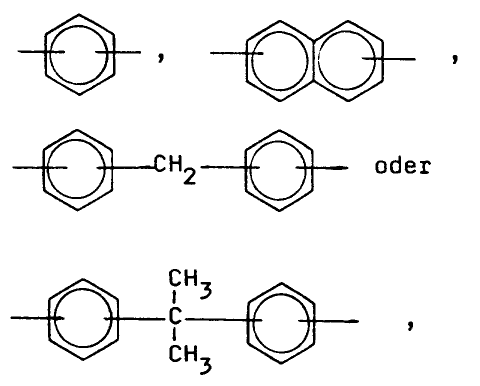 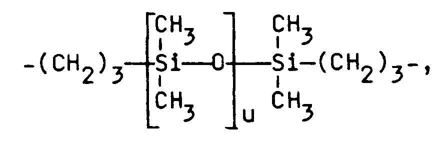 mit u = 0 bis 150,
oder die entsprechende Gruppierung aus 3,4-Epoxycyclohexylmethyl-3',4'-epoxycyclohexancarboxylat;
R² = -(CH₂-CH=CH-CH₂)ₙ- , -R⁶- , -R⁶-O-CO-R⁷-CO-O-R⁶- oder 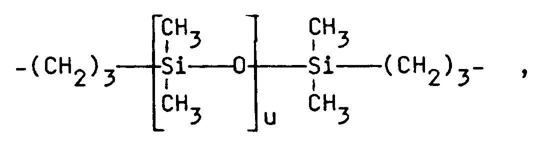 wobei n = 1 bis 50, u = 0 bis 150,
R⁶ dieselbe Bedeutung hat wie R⁵,
außer 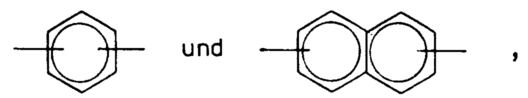 und
R⁷ folgendes bedeutet:
-(CH₂)ᵥ-, 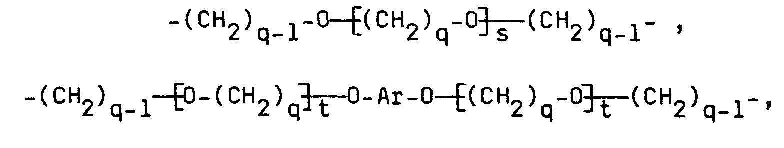 mit q = 2 bis 4, s = 0 bis 50, t = 0 bis 25,
v = 0 bis 20, und
Ar die vorstehend angegebene Bedeutung hat.
